# EUROPEAN PATENT APPLICATION

(11) **EP 1 548 103 A1**
(43) Date of publication of application: **29.06.2005**
(21) Application number: 03792746.4
(22) Date of filing: 20.08.2003
(51) Int. Cl.: C12N 15/00, C12Q 1/68, C12M 1/00

(54) **HYBRIDIZATION SENSING PART, SENSOR CHIP, AND HYBRIDIZATION METHOD**

(30) Priority: 20.08.2002 JP 2002239642; 20.08.2002 JP 2002239643; 08.10.2002 JP 2002294796; 15.10.2002 JP 2002300924
(71) Applicant: Sony Corporation, Tokyo 141-0001 (JP)
(72) Inventor: SEGAWA, Yuji, Shinagawa-ku, Tokyo 141-0001 (JP); MAMINE, Takayoshi, Shinagawa-ku, Tokyo 141-0001 (JP); SAKAMOTO, Yasuhiro, Shinagawa-ku, Tokyo 141-0001 (JP); YOSHIO, Akira, Shinagawa-ku, Tokyo 141-0001 (JP); YAMAMOTO, Takuro, Shinagawa-ku, Tokyo 141-0001 (JP)
(74) Representative: Mills, Julia
(86) International application number: PCT/JP2003/010523
(87) International publication number: WO 2004/018663

(57) **Abstract**

The present invention provides a hybridization detector (1a) and the like that are improved in the hybridization efficiency by arraying nucleotide probes in a stretched form, a sensor chip including the hybridization detector (1a) or the like, and a method of hybridization using these detectors or the chip. The hybridization detector (1a) and the like include a reaction region (R) for hybridization between the nucleotide probes (X) and target nucleotide sequences (Y) having a base sequence complementary to the nucleotide probes (X). The reaction region (R) has a configuration for stretching the nucleotide probes (X) by an electric field and immobilizing the nucleotide probes (X) on ends (E) of scanning electrodes (C) by dielectrophoresis.

## Description

### Technical Field

The present invention relates to a technology used in hybridization detectors that are suitable for sensor chips such as a DNA chip. In particular, the present invention relates to an improvement in hybridization efficiency by arraying and immobilizing nucleotide probes in a stretched form on predetermined portions of electrodes in reaction regions where hybridizations are performed.

### Background Art

Main conventional technologies related to the present invention will now be described. Currently, a slide, called a DNA chip or a DNA microarray (collectively referred to as "DNA chip", hereinafter), on which selected DNAs are finely arrayed by a microarray technology, is used for bioassay to analyze gene variants, single nucleotide polymorphisms (SNPs), gene expression frequency, and so on. The DNA chip has been widely used in the field of drug discovery, clinical diagnosis, pharmacological genomics, forensic medicine, and the like.

The DNA chip, which is formed by arraying various and many oligo DNAs, complementary DNAs (cDNAs), or the like on a glass substrate or on a silicon substrate, is characteristically capable of analyzing exhaustively molecule interaction by hybridization.

A brief explanation of an analysis using the DNA chip is as follows: mRNAs extracted from cells, tissues, or the like are used for amplification by a polymerase chain reaction (PCR) such as a reverse transcription-PCR incorporating fluorescence-labeled dNTP; the resulting cDNAs are hybridized to DNA probes immobilized on the glass substrate or the silicon substrate; and then the fluorescence on the substrate is measured by a predetermined detecting device.

The DNA chip is classified into two types. The first type is made by directly synthesizing oligonucleotides on a predetermined substrate using photolithographic techniques combined with a light exposure process for manufacturing semiconductors. Affymetrix Inc. is a pioneer of this technology (see, for example, PCT Japanese Translation Patent Publication No. 4-505763). In the DNA chips classified in this type, DNAs are densely arrayed but the length of the DNAs synthesized on the substrate is limited to a span of several tens of bases.

The second type, which is called "Stanford-style", is made by dispensing and immobilizing pre-synthesized DNAs on a substrate with a split pin (see, for example, PCT Japanese Translation Patent Publication No. 10-503841). The DNA chips of this type has a low density of arrayed DNAs than that of the first type, but can immobilize as much as 1 kb of a DNA fragment.

IEEE TRANSACTIONS ON INDUSTRY APPLICATIONS, VOL. 31, No. 3, MAY/JUNE 1995, p. 451 discloses a technique for immobilizing DNAs on electrode surfaces (DNA immobilization method) by electric fields generated by floating-potential electrodes aligned between counter electrodes.

Nucleotide probes, i.e. DNA probes immobilized on a detection surface (spotted portion), fabricated by the above-mentioned conventional DNA chip technologies are randomly coiled or folded by Brownian motion and are unevenly arrayed on the detection surface.

Consequently, target nucleotide sequences are sterically hindered from hybridization. As a result, hybridization shows the following technical disadvantages: low efficiency, a longer hybridization time, and possibility of false positivity or false negativity.

To improve the hybridization efficiency, the DNA immobilization method described in IEEE TRANSACTIONS ON INDUSTRY APPLICATIONS, VOL. 31, No. 3, MAY/JUNE 1995, p. 451 can be applied for immobilization of DNAs in a stretched form on a detection surface (spotted portion) of a known DNA chip, which will decrease the steric hindrance. Namely, counter electrodes and floating-potential electrodes aligned between the counter electrodes are arranged on the detection surface of the DNA chip, and electric fields are applied to a nucleotide solution so that DNAs are immobilized on the surfaces of the floating-potential electrodes. However, according to this DNA immobilization method, since the floating-potential electrodes having the same length as those of the counter electrodes are merely aligned between the counter electrodes, the magnitude and the density of non-uniform electric fields generated on the surfaces of the floating-potential electrodes are low. Therefore, the amount of the immobilized DNAs is insufficient. Accordingly, it is predicted that the hybridization efficiency is not sufficiently improved.

It is a main object of the present invention to improve the hybridization efficiency by arraying and immobilizing nucleotide probes in a stretched form on detection surfaces in reaction regions where hybridizations are performed.

### Disclosure of Invention

In order to resolve the above-mentioned technological problems, the present invention provides a hybridization detector and a sensor chip including the hybridization detector and also provides a hybridization method using the hybridization detector or the sensor chip. The hybridization detector includes a reaction region for hybridization between nucleotide probes and target nucleotide sequences having a base sequence complementary to the nucleotide probes. The reaction region has a configuration for stretching the nucleotide probes by an electric field and immobilizing the nucleotide probes by dielectrophoresis on scanning electrodes arrayed in the reaction region.

The present invention provides the following effects: The nucleotide probes can be stretched by the electric field generated in the reaction region; a non-uniform electric field (an electric field of which electric lines of force are partially concentrated) is generated in the vicinity of ends of the scanning electrodes arrayed in the reaction region; the stretched nucleotide probes in the reaction region can migrate toward the ends of the scanning electrodes by dielectrophoresis due to the non-uniform electric field; and the nucleotide probes in a stretched form can be immobilized on the scanning electrodes so as to bridge between the electrodes.

The configuration of the electrodes (including a counter electrode and a common electrode) for generating an electric field to stretch nucleotide sequences is appropriately determined so as to provide the above-mentioned effects, and the configuration of the scanning electrodes for immobilizing the stretched nucleotide probes is also appropriately determined so as to provide the above-mentioned effects. Regarding the scanning electrodes consisting of an appropriate number of electrodes, electrodes are sequentially energized by switching on and off switches disposed at a predetermined place of a chip, the nucleotide probes (in a stretched form) near the energized electrodes (i.e. electrodes to which a voltage is applied) migrate toward the electrodes by dielectrophoresis, and the nucleotide probes can be immobilized one after the other so as to bridge each end of the adjacent scanning electrodes.

The nucleotide probes, which are dropwise added to and dispersed in the reaction region, are entangled in a randomly coiled form (which is unsuitable for hybridization) by Brownian motion. The nucleotide probes are arranged into a stretched form suitable for hybridization, and are then arrayed and immobilized in a stretched form on the scanning electrodes by the above-mentioned means or method. Consequently, the density of the nucleotide probes can be made uniform in the reaction region.

Target nucleotide sequences, which are subsequently added to the reaction region, can be stretched and migrate toward the scanning electrodes in the same manner as the nucleotide probes and can be hybridized to the nucleotide probes arrayed and immobilized in the reaction region. Since bases are not folded in the stretched nucleotide probes nor in the stretched target nucleotide sequences, the hybridization can be smoothly performed without steric hindrance.

A nucleotide sequence is stretched based on the following principle: when a high-frequency electric field of about 1 MV/m is applied to the nucleotide sequence (which includes a large number of polarization vectors having a negative charge of a phosphate ion or the like of the nucleotide sequence and a positive charge of an ionized hydrogen atom), dielectric polarization occurs in the nucleotide sequence. As a result, the nucleotide molecule is stretched in parallel with the electric field.

Furthermore, in order to resolve the above-mentioned technological problems, the present invention provides a hybridization detector including a reaction region for hybridization between nucleotide probes and target nucleotide sequences having a base sequence complementary to the nucleotide probes, counter electrodes disposed at the reaction region, and a plurality of floating-potential electrodes distributed between the counter electrodes.

The alignment of the counter electrodes is not limited. Preferably, the counter electrodes are aligned in parallel with each other at the reaction region because such an alignment can generate a uniform high-density electric field over the entire reaction region between the counter electrodes. As a result, each of the nucleotide sequences in the reaction region can be stretched along the electric lines of force.

A nucleotide sequence is stretched based on the following principle: when a high-frequency electric field of about 1 MV/m is applied to the nucleotide sequence (which includes a large number of polarization vectors having a negative charge of a phosphate ion or the like of the nucleotide sequence and a positive charge of an ionized hydrogen atom), dielectric polarization occurs in the nucleotide sequence. As a result, the nucleotide molecular is stretched in parallel with the electric field.

Bases, not folded in the stretched nucleotide probes nor in the stretched target nucleotide sequences, can be readily hybridized without steric hindrance. Therefore, the probability of association (a hydrogen bond) between complementary base pairs can be increased and hybridization between nucleotide sequences located near each other can be smoothly performed.

In the present invention, the floating-potential electrodes can generate a large number of areas where the electric lines of force are partially concentrated in the reaction region, and immobilize (the ends of) the nucleotide probes to the areas. The floating-potential electrodes distributed in the reaction region between the counter electrodes can uniformly generate areas where the electric lines of force are concentrated at a high density in the reaction region. Furthermore, the reaction region including the distributed floating-potential electrodes allows the nucleotide sequences to freely migrate in the reaction region.

The shape of the floating-potential electrodes is not limited, however, from the viewpoint of readily concentrating electric fields (electric lines of force) and readily immobilizing nucleotide sequences, a circular or polygonal shape is preferable. The floating-potential electrodes formed into a circular or polygonal shape are beneficial for generating a non-uniform electric field. The surface of the floating-potential electrode is preferably smaller than that of the counter electrode because the electric fields can be readily concentrated toward the electrode surfaces of the floating-potential electrodes. Consequently, non-uniform electric fields can be readily generated. Furthermore, the nucleotide probes can be surely immobilized by treating the surfaces of the floating-potential electrode so as to immobilize the nucleotide probes.

The present invention provides a sensor chip including the above-mentioned hybridization detector. More particularly, the present invention provides a sensor chip including a reaction region for hybridization between nucleotide probes and target nucleotide sequences having a base sequence complementary to the nucleotide probes, counter electrodes disposed at the reaction region, and a plurality of floating-potential electrodes aligned between the counter electrodes. The sensor chip can stretch the nucleotide probes in the reaction region by applying a voltage to the counter electrodes and immobilize the stretched nucleotide probes on the surfaces of the counter electrodes and the floating-potential electrodes by dielectrophoresis in non-uniform electric fields generated at the counter electrodes and at the partial surfaces of the floating-potential electrodes.

The sensor chip enables the arrangement of the nucleotide probes, which are dropwise added to and are dispersed in the reaction region and are entangled in a randomly coiled form (which is unsuitable for hybridization) by Brownian motion, to a stretched form suitable for hybridization and enables the immobilization of the nucleotide probes in a stretched form on the surfaces of the floating-potential electrodes.

The target nucleotide probes in the reaction region can be stretched by applying a voltage to the counter electrodes and be hybridized to the nucleotide probes immobilized on the surfaces of the counter electrodes and the floating-potential electrodes. Preferably, the electric fields generated by the counter electrodes of the sensor chip according to the present invention are of alternate current.

The present invention provides a method of hybridization. The method uses a detection surface including a reaction region for hybridization between nucleotide probes and target nucleotide sequences having a base sequence complementary to the nucleotide probes, counter electrodes disposed at the reaction region, and a plurality of floating-potential electrodes aligned between the counter electrodes.

The method includes the steps of: immobilizing (a first step) the nucleotide probes on the surfaces of the floating-potential electrodes by stretching the nucleotide probes in the reaction region by applying a voltage to the counter electrodes, and by dielectrophoresis of the stretched nucleotide probes in non-uniform electric fields generated at the counter electrode and at the partial surfaces of the floating-potential electrodes; and hybridizing (a second step) the target nucleotide sequences to the nucleotide probes immobilized on the surfaces of floating-potential electrodes in a stretched form by stretching the target nucleotide sequences in the reaction region by applying a voltage to the counter electrodes.

In this method, the voltage applied to the counter electrodes generates electric fields in the reaction region, i.e. non-uniform electric fields are generated at the surfaces of the floating-potential electrodes aligned in the reaction region and the surfaces of the counter electrodes. The nucleotide probes are immobilized on the surfaces of the electrodes in a stretched form by dielectrophoresis in the non-uniform electric fields in the first step.

In the second step, the target nucleotide sequences are subsequently added to the reaction region. The target nucleotide sequences are stretched and migrate toward the nucleotide probes, which are immobilized on the surfaces in a stretched form, by dielectrophoresis in the same manner as above. The hybridization can be efficiently performed between the nucleotide probes in a stretched form and the target nucleotide sequences in a stretched form.

The present invention has a technological advantage in the field of drug discovery, clinical diagnosis, pharmacological genomics, forensic medicine, and other related industries. Namely, the present invention provides a hybridization detector and a sensor chip such as a DNA chip including the detector that can efficiently detect the hybridization required for analysis of gene variants, single nucleotide polymorphisms (SNPs), gene expression frequency, and so on.

The definition of main technical terms in the present invention will now be described below. In the present invention, "nucleotide sequence" means a polymer of a phosphate ester of a nucleoside that is a purine or pyrimidine base in glycosidic linkage with a sugar. Oligonucleotides including DNA probes, polynucleotides, DNAs (full length or partial length) being a polymerized form of a purine nucleotide and a pyrimidine nucleotide, resulting cDNAs (cDNA probes) of reverse-transcription, RNAs, and polyamide nucleotide analogs (PNA) are included.

"Nucleotide probe" is a nucleotide sequence that is directly or indirectly immobilized on the detection surface. "Target nucleotide sequence" is a nucleotide sequence that has a base sequence complementary to the nucleotide probe and, in some cases, is labeled with a fluorescent material or the like.

"Hybridization" means a duplex (double-strand) formation reaction between nucleotide sequences having a complementary base sequence.

"Reaction region" is a reservoir of nucleotide solutions and is a region for a hybridization reaction in a liquid phase. In the reaction region, other interactions in addition to single-stranded nucleotide interactions or hybridizations can proceed: interactions between a peptide (or a protein) and double-stranded nucleotide formed according to the nucleotide probe, and molecular interactions such as an enzyme reaction. For example, when a double-stranded nucleotide is used, the binding between a receptor molecule such as a hormone receptor acting as a transcription factor and the corresponding sequence of DNA can be analyzed.

"Steric hindrance" means a phenomenon in which a bulky substituent near the reaction center of a molecule or a conformation or steric structure (higher-order structure) of a reaction molecule inhibits a reaction partner from approaching. Consequently, a desired reaction (hybridization in the present invention) is hindered.

"Dielectrophoresis" is a phenomenon in which a molecule migrates to a higher electric field in a non-uniform electric field. Even when an alternate current is applied, since the polarity of polarization is inverted according to the inversion of the polarity of the voltage, the migration proceeds in the same manner as that with the application of a direct current (see edited by Teru Hayashi, "Maikuromashine to Zairyo Gijutsu (Micro-machine and Material Technology)", CMC books, pp. 37-46, Chapter 5: Manipulation of Cells and DNA).

"Counter electrodes" are formed of at least a pair of opposing electrodes in the reaction region. The counter electrodes generate an electric field in the reaction region when a voltage is applied to them.

"Common electrode" means an electrode from which a voltage can be applied to a plurality of electrodes. "Scanning electrode" means a group of arrayed electrodes to which a voltage can be sequentially applied by switching on and off.

"Floating-potential electrode" means an isolated electrode that is conductive and is not connected to an outer power source.

"Sensor chip" means a substrate, which is composed of quartz glass, synthetic resin, or the like, including the reaction region and the detection surface for detecting a molecular interaction. A typical example of the sensor chip is a DNA chip.

### Brief Description of the Drawings

Fig. 1 illustrates a main configuration (1a) of a hybridization detector or a sensor chip of the present invention.
Fig. 2A shows a cross-sectional view of the detector (1a) shown in Fig. 1 taken along line I-I and viewed from the direction of arrows I. Fig. 2B shows a cross-sectional view of a modification (1'a) of the detector (1a) shown in Fig. 2A taken along line I-I and viewed from the direction of arrows I.
Fig. 3 shows a non-uniform electric field L₂ generated in the vicinity of scanning electrodes Cx and Cy of the detector 1a (or the detector 1'a) by applying a voltage between counter electrodes A and B, between second counter electrodes G and Cx, and between the second counter electrodes G and Cy.
Fig. 4 illustrates a main configuration (reference numeral: 1b) of a detector or a sensor chip according to a second embodiment of the present invention.
Fig. 5 illustrates a main configuration (reference numeral: 1c) of a detector or a sensor chip according to a third embodiment of the present invention.
Fig. 6 illustrates a main configuration (reference numeral: 1d) of a detector or a sensor chip according to a fourth embodiment of the present invention.
Figs. 7A to 7C show voltage-applying operations for the detector 1a (three examples).
Figs. 8A to 8C show examples of the voltage-applying operations for the detector 1b (three examples).
Fig. 9 illustrates a main configuration (reference numeral: 1e) of a detector or a sensor chip according to a fifth embodiment of the present invention.
Fig. 10 illustrates a main configuration (reference numeral: 1f) of a detector or a sensor chip according to a sixth embodiment of the present invention.
Fig. 11 illustrates a main configuration (reference numeral: 1g) of a detector or a sensor chip according to a seventh embodiment of the present invention.
Fig. 12A shows a cross-sectional view taken along line II-II and viewed from the direction of arrows II in Fig. 9. Fig. 12B shows a cross-sectional view of a modification (reference numeral: 1'e) of the detector (1e) taken along line II-II and viewed from the direction of arrows II.
Fig. 13A shows examples of the voltage-applying operations for the detector (1e), Fig. 13B shows examples of the voltage-applying operations for the detector (1f), and Fig. 13C shows examples of the voltage-applying operations for the detector (1g).
Fig. 14 shows another example of the voltage-applying operation.
Fig. 15 illustrates a main configuration (reference numeral: 1h) of a detector or a sensor chip according to an eighth embodiment of the present invention.
Fig. 16 illustrates a main configuration (reference numeral: 1i) of a detector or a sensor chip according to a ninth embodiment of the present invention.
Fig. 17 illustrates the main configuration (reference numeral: 1i) according to the ninth embodiment.
Fig. 18A shows a cross-sectional view taken along line III-III and viewed from the direction of arrows III in Figs. 16 and 17, and Fig. 18B shows a cross-sectional view of a modification (reference numeral: 1'i) of the detector 1i shown in Fig. 18A taken along line III-III and viewed from the direction of arrows III.
Fig. 19A illustrates scanning electrodes having a rectangular end (Ea), Fig. 19B illustrates scanning electrodes having a triangular end (Eb), and Fig. 19C illustrates scanning electrodes having a circular end (Ec).
Fig. 20 illustrates a main configuration (10a) of a detector or a sensor chip according to a tenth embodiment of the present invention.
Fig. 21 shows a non-uniform electric field (L) generated in the detector.
Fig. 22 illustrates a main configuration (10b) of a detector or a sensor chip according to an eleventh embodiment of the present invention.
Fig. 23 is a brief flowchart showing a process of a method of hybridization of the present invention.

### Best Mode for Carrying Out the Invention

Preferred embodiments of the present invention will now be described with reference to the accompanying drawings.

### [First Embodiment]

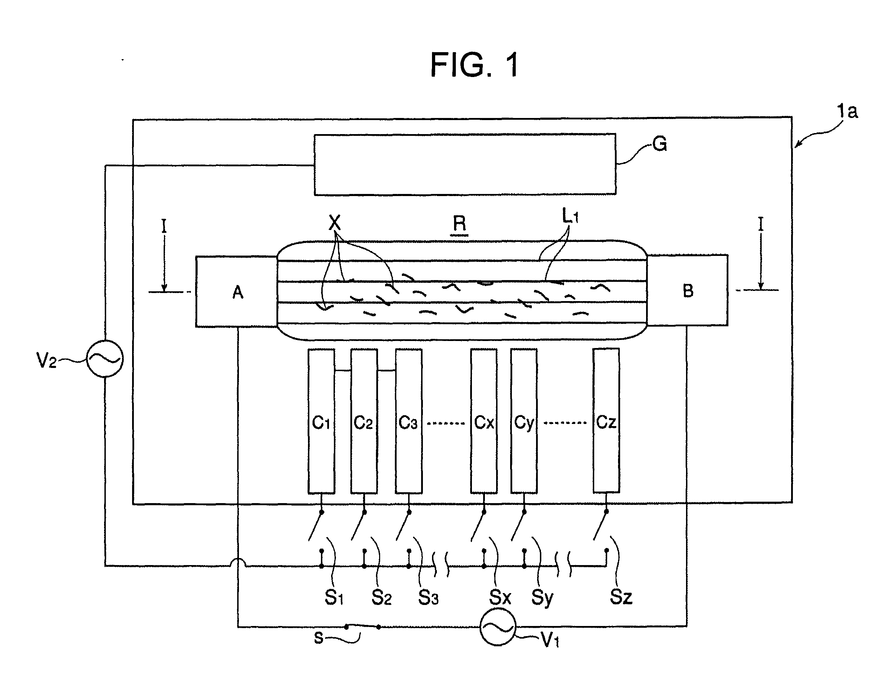

Fig. 1 illustrates a main configuration (reference numeral: 1a) of a hybridization detector (abbreviated to "detector" hereinafter) or a sensor chip according to a first embodiment of the present invention.

The detector 1a according to the first embodiment has a reaction region R. The reaction region R is provided in a reservoir for adding a nucleotide solution containing nucleotide probes X or target nucleotide sequences Y, and the reaction region R is a portion or space for hybridizations are performed.

Counter electrodes A and B are disposed at the reaction region R and are connected to a power source V₁. Preferably, the counter electrodes A and B are aligned in parallel with each other. When a high-frequency voltage is applied from the power source V₁ by switching on a switch s shown in the drawing, a uniform electric field (which is an electric field in which electric lines of force are not concentrated in one part) is generated in the reaction region R between the counter electrodes A and B (see lines denoted by a reference numeral L₁ in Fig. 1).

Preferably, the parameters of the electric field between the counter electrodes A and B are about 1 × 10⁶ V/m and about 1 MHz (see Masao Washizu and Osamu Kurosawa: "Electrostatic Manipulation of DNA in Microfabricated Structures", IEEE Transaction on Industrial Application Vol. 26, No. 26, p.1165-1172 (1990)). Since all the parameters of the electric field described below are the same as these parameters, the explanation will be omitted hereinafter.

The uniform electric field L₁ stretches the nucleotide probes X, which are dispersed in the reaction region R in a random coil state or the like, to a stretched form along the uniform electric field L₁ (the principle is already described).

Second counter electrodes G and C (consisting of C₁, C₂, C₃, ..., Cx, Cy, and Cz) are disposed between the counter electrodes A and B so as to be orthogonal to the counter electrodes A and B. Each of the second counter electrodes is connected or can be connected to a power source V₂ shown in the drawing.

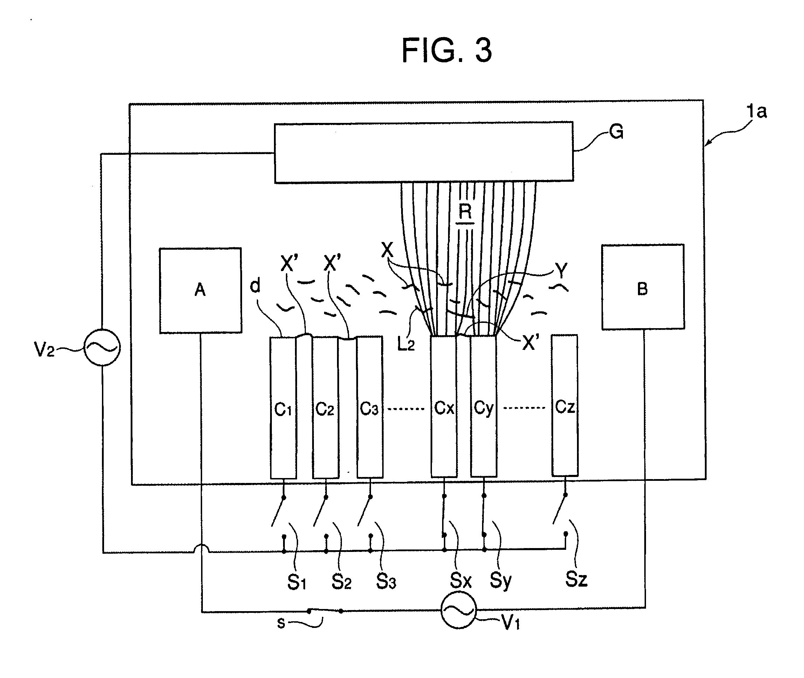

In the detector 1a, the electrode G is a single rectangular electrode, and the electrodes C are scanning electrodes. Each of the scanning electrodes is aligned to oppose the electrode G with a predetermined distance. In the scanning electrodes C, a voltage is applied to a pair of adjacent scanning electrodes D one after the other by switching on and off switches S₁, S₂, S₃, ..., Sx, Sy, and Sz one by one. Electrical connection generates a non-uniform electric field L₂ between the scanning electrodes (for example, between G and Cx and between G and Cy). In the non-uniform electric field, electric lines of force are concentrated partially (especially in the vicinity of the ends of scanning electrodes C) as shown in Fig. 3.

Each space between the electrodes C₁, C₂, ..., and Cz in the scanning electrodes C is smaller than a molecular length of the nucleotide probes X' so that the scanning electrodes are bridged by the nucleotide probes X' in a stretched form (the same is applied to all embodiments hereinafter).

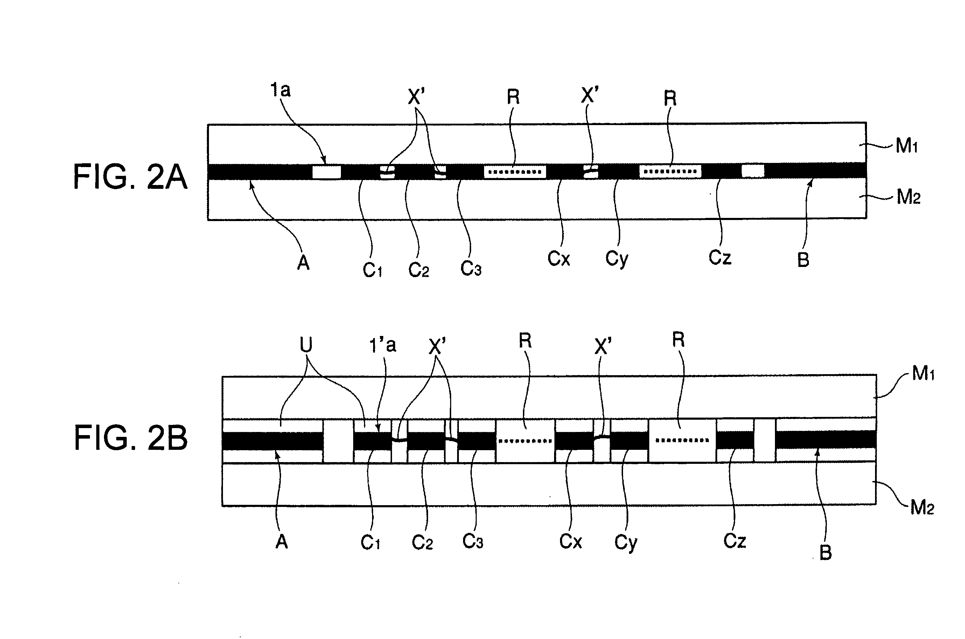

Fig. 2A shows a cross-sectional view taken along line I-I and viewed from the direction of arrows I in Fig. 1. Fig. 2B shows a cross-sectional view of a modification (reference numeral: 1'a) of the detector 1a shown in Fig. 2A taken along line I-I and viewed from the direction of arrows I.

As shown in Fig. 2A, the detectors 1a and the modified detector 1'a are disposed in a narrow gap between substrates M₁ and M₂ composed of quartz glass, silicon, or a synthetic resin such as polycarbonate or polystyrene. In the detector 1a, the depth (or width) of the reaction region R is the same as the thicknesses of the electrodes such as the counter electrodes A and B.

In some cases represented by the detector 1'a shown in Fig. 2B, dielectric layers U may be disposed so as to sandwich the counter electrodes A and B and the rest. In this configuration, the gap between the substrates M₁ and M₂ is increased and the capacity of the reaction region R is increased. In the reaction region R, the scanning electrodes C may be disposed in a plurality of lines in the depth direction of the reaction region R (not shown).

Fig. 3 shows a non-uniform electric field L₂ generated in the vicinity of the scanning electrodes Cx and Cy of the detector 1a (or the detector 1'a) by applying a voltage between the counter electrodes A and B, between the second counter electrodes G and Cx, and between the second counter electrodes G and Cy. In Fig. 3, target nucleotide sequences Y are already added to the reaction region R. An electric field (L₁) generated between the counter electrodes A and B is not shown.

As shown in Fig. 3, the nucleotide probes X, which are stretched by the uniform electric field generated between the counter electrodes A and B, migrate toward the gaps between adjacent scanning electrodes C₁ and C₂, C₂ and C₃, ..., Cx and Cy, ... by dielectrophoresis to be immobilized on the ends d of the electrodes D and to bridge the ends d and d.

The reference numeral X' in Fig. 3 denotes the nucleotide probes immobilized between the adjacent scanning electrodes C (the same is applied to other drawings). In Fig. 3, switches Sx and Sy are switched on and non-uniform electric fields L₂ are generated between the electrodes G and Cx and between G and Cy.

The target nucleotide sequences Y, which are subsequently added to the reaction region R, are stretched by the uniform electric field between the counter electrodes A and B in the same manner as the nucleotide probes X. The stretched target nucleotide sequences Y migrate toward the nucleotide probes X' (in a stretched form) immobilized between the adjacent electrodes C, and the complementary sequence portions of the target nucleotide sequences are hybridized to the nucleotide probes efficiently without steric hindrance.

### [Second Embodiment]

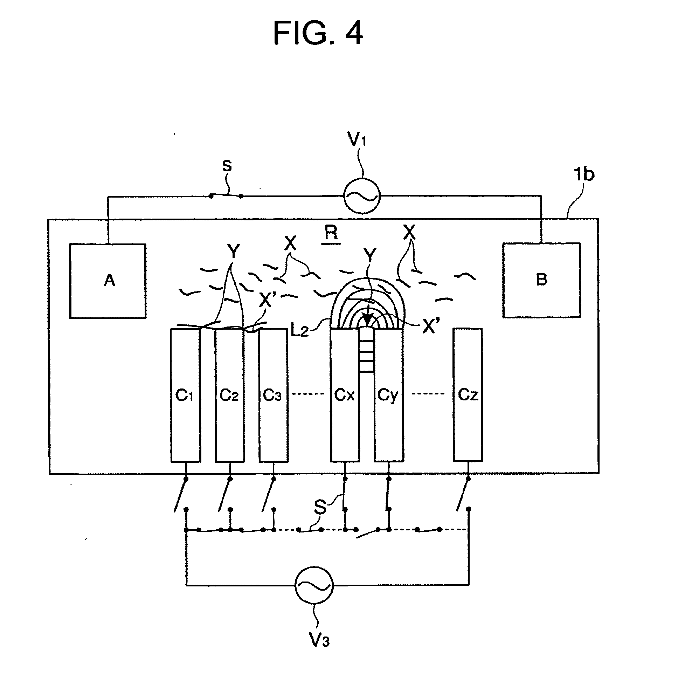

Fig. 4 illustrates a main configuration (reference numeral: 1b) of a detector or a sensor chip according to a second embodiment of the present invention.

The detector 1b differs from the detectors 1a and 1'a of the first embodiment in that the detector 1b does not have the electrode (denoted by reference numeral G in Figs. 1 and 3) opposing to the scanning electrodes C₁, C₂, C₃, ..., Cx, Cy, and Cz. A power source V₃ shown in the drawing applies a voltage between the adjacent scanning electrodes C1 and C2, C2 and C3, ..., Cx and Cy, ... one after the other by switching on and off the corresponding switches S shown in the drawing according to a predetermined procedure.

In Fig. 4, voltages are applied between the counter electrodes A and B and between the scanning electrodes Cx and Cy to generate a non-uniform electric field L₂ between the scanning electrodes Cx and Cy. The target nucleotide sequences Y migrate toward the nucleotide probes X' immobilized between the scanning electrodes Cx and Cy (see a region indicated by an arrow).

### [Third Embodiment]

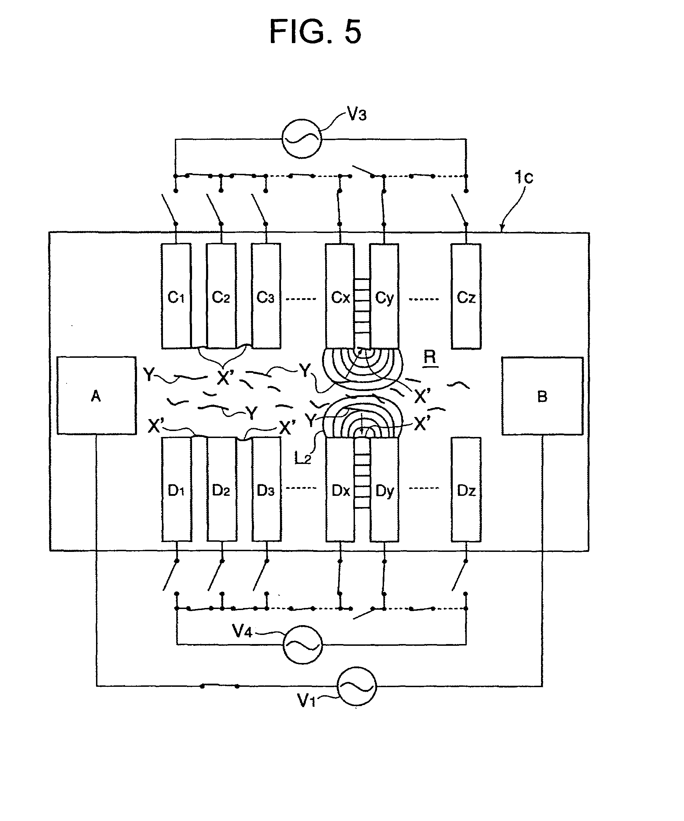

Fig. 5 illustrates a configuration (reference numeral: 1c) of a detector or a sensor chip according to a third embodiment of the present invention.

The detector 1c shown in Fig. 5 has scanning electrodes C and scanning electrodes D between the counter electrodes A and B. The scanning electrodes C are energized by a power source V₃ and the scanning electrodes D energized by a power source V₄. Both the scanning electrodes C and the scanning electrodes D oppose each other. Specifically, the scanning electrodes C₁, C₂, C₃, ..., Cx, Cy, and Cz, which are collaterally disposed, are aligned so as to oppose the scanning electrodes D₁, D₂, D₃, ..., Dx, Dy, and Dz, respectively.

In Fig. 5, voltages are applied between the counter electrodes A and B, between the scanning electrodes Cx and Cy, and between the scanning electrodes Dx and Dy. The target nucleotide sequences Y migrate toward the nucleotide probes X' immobilized between the scanning electrodes Cx and Cy and between the scanning electrodes Dx and Dy by the non-uniform electric fields L₂ (see regions indicated by arrows).

### [Fourth Embodiment]

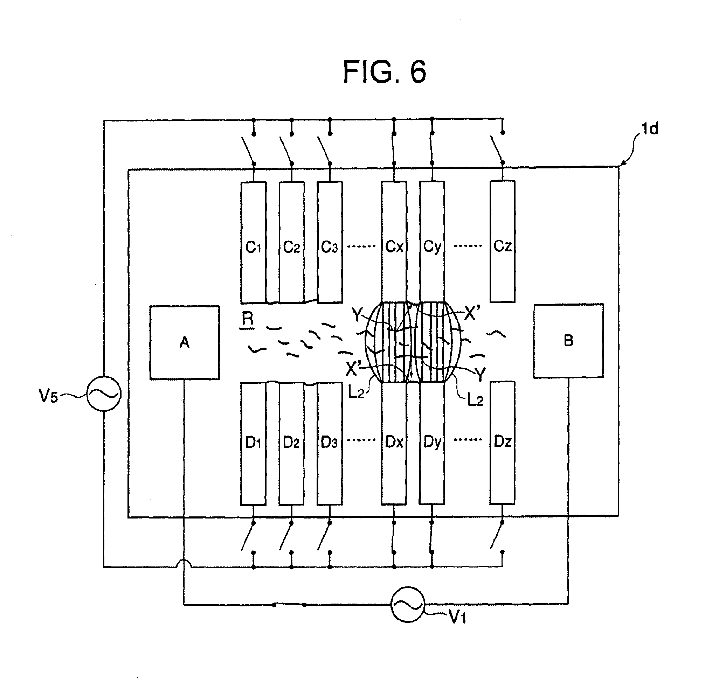

Fig. 6 illustrates a main configuration (reference numeral: 1d) of a detector or a sensor chip according to a fourth embodiment of the present invention.

The detector 1d is the same as the detector 1c according to the third embodiment in that the scanning electrodes C₁, C₂, C₃, ..., Cx, Cy, and Cz, which are collaterally disposed, are aligned so as to oppose the scanning electrodes D₁, D₂, D₃, ..., Dx, Dy, and Dz, respectively. However, in the detector 1d, a common power source V₅ applies a voltage to both the scanning electrodes C and the scanning electrodes D.

In Fig. 6, voltages are applied between the counter electrodes A and B, between the scanning electrodes Cx and Dx, and between the scanning electrodes Cy and Dy. The target nucleotide sequences Y migrate toward the immobilized nucleotide probes X' by the non-uniform electric fields L₂ (see regions indicated by arrows).

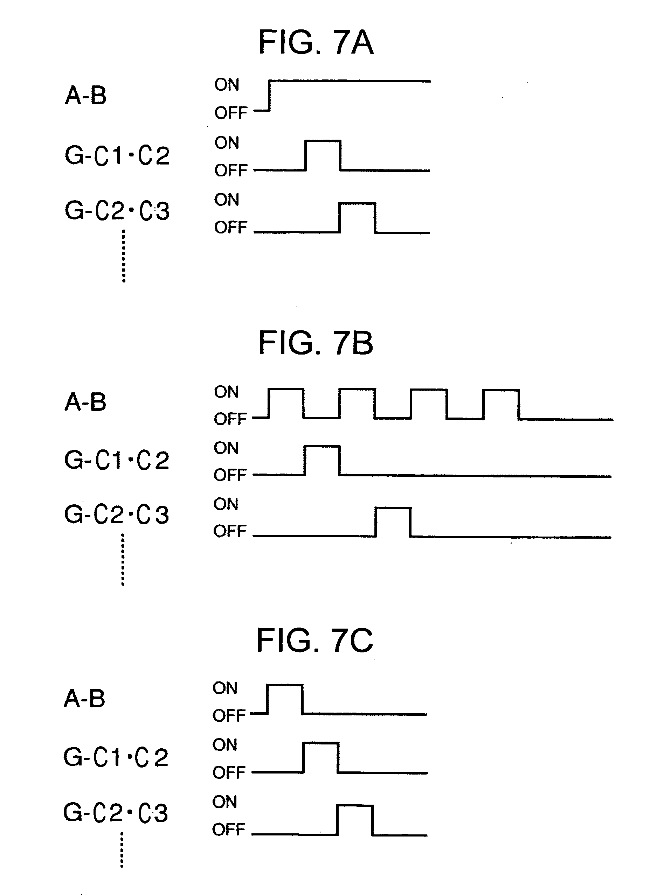

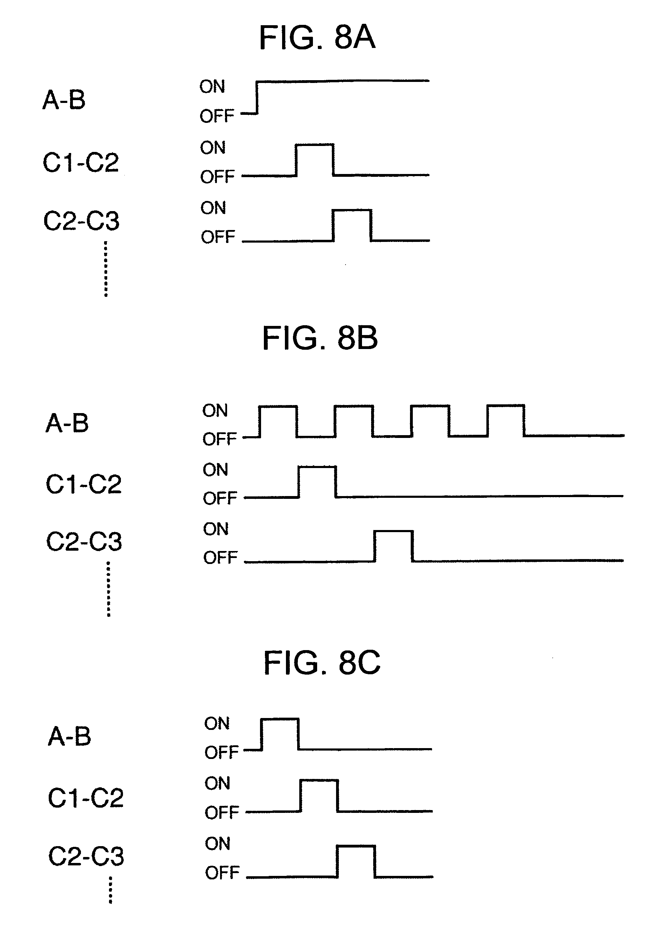

Voltage-applying operations will be explained with reference to Figs. 7A, 7B, and 7C and Figs. 8A, 8B, and 8C. Figs. 7A to 7C show examples of the voltage-applying operations of the detector 1a (three examples) and Figs. 8A to 8C show examples of the voltage-applying operations of the detector 1b (three examples).

For example, in the detector 1a shown in Fig. 1, as shown in Fig. 7A, the voltage may be constantly applied between the counter electrodes A and B when the scanning electrodes are switched on in a sequential order ((G and C₁) and (G and C₂) → (G and C₂) and (G and C₃) → ...).

As shown in Fig. 7B, the voltage applied between the counter electrodes A and B may be discontinued every time each of the scanning electrodes is switched on one after the other.

As shown in Fig. 7C, the voltage applied between the counter electrodes A and B may be discontinued while a voltage is applied to the scanning electrodes. The repetition of this voltage-applying operation allows nucleotide probes X to be securely immobilized on the scanning electrodes.

In the detector 1b shown in Fig. 4, as shown in Fig. 8A, the voltage may be constantly applied between the counter electrodes A and B when the scanning electrodes are switched on one after the other (C₁ and C₂ → C₂ and C₃ → ...).

As shown in Fig. 8B, the voltage applied between the counter electrodes A and B may be discontinued every time each of the scanning electrodes is switched on one after the other.

As shown in Fig. 8C, the voltage applied between the counter electrodes A and B may be discontinued while a voltage is applied to the scanning electrodes. The repetition of this voltage-applying operation allows nucleotide probes X to be securely immobilized on the scanning electrodes. The voltage-applying operation can also be conducted for the detector 1c and the detector 1d.

As shown in Figs. 7B, 7C, 8B, and 8C, application of an intermittent voltage between the counter electrodes A and B has advantages: the target nucleotide sequences Y in the reaction region R can migrate stepwise to the immobilized nucleotide probes X', the target nucleotide sequences Y can migrate backward and forward, and the reaction timing can be controlled.

When the voltage application between the counter electrodes A and B is discontinued, the duplex-formation reaction, i.e. hybridization, between the target nucleotide sequences Y in a stretched form and the nucleotide probes X' in a stretched form proceeds merely under Brownian motion.

The above-mentioned voltage-applying operations allow a hydrogen bond between the nucleotide probes X' immobilized on the scanning electrodes D in a stretched form and complementary bases of the target nucleotide sequences Y in a stretched form in the same manner as the nucleotide probes to be efficiently formed without steric hindrance.

Namely, the nucleotide probes X' and the target nucleotide sequences Y are efficiently hybridized. As a result, the hybridization time can be decreased and the probability of false positivity or false negativity is reduced.

### [Fifth Embodiment]

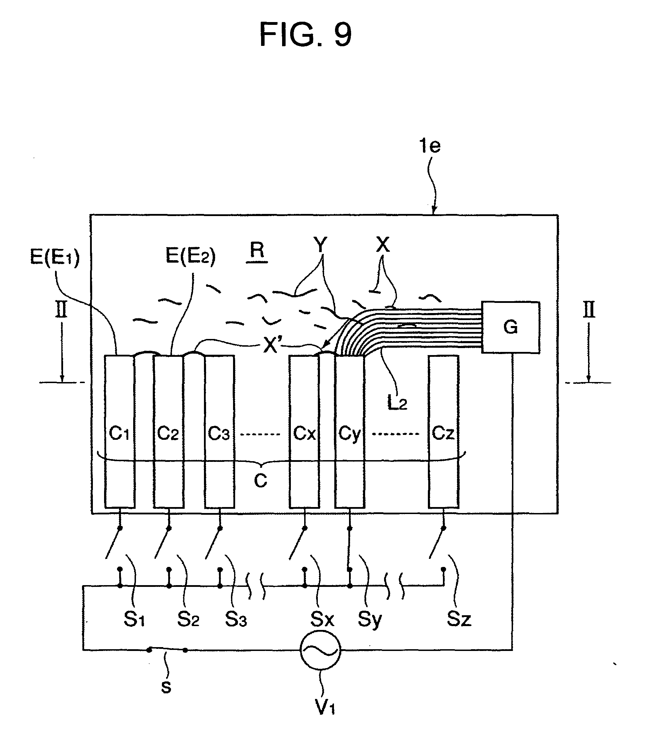

Fig. 9 illustrates a main configuration (reference numeral: 1e) of a detector or a sensor chip according to a fifth embodiment of the present invention.

The detector denoted by reference numeral 1e has a reaction region R. In the reaction region R, a common electrode G and scanning electrodes C (consisting of C₁ to Cz) aligned in parallel with the common electrode G are disposed.

The common electrode G is connected to a power source V₁. Each electrode C₁ to Cz of the scanning electrodes C aligned in a line is connected to the power source V₁ by switching on the switches S₁ to Sz one after the other. Fig. 9 shows a state (stage): a switch Sy is switched on, a voltage is applied between the electrodes G and Cy to generate a non-uniform electric field L₂ in the vicinity of the scanning electrode Cy.

By switching on and off switches S₁ to Sz one after the other for applying a high-frequency voltage between the common electrode G and the electrodes C₁ to Cz of the scanning electrodes C one by one, a non-uniform electric field L₂ is generated between the electrode G and each of the electrodes C₁ to Cz in the reaction region R.

The electric field stretches the nucleotide probes X, which are dispersed in the reaction region R in a random coil state or the like, to be linearly arranged along the electric field, and, at the same time, the stretched nucleotide probes X migrate toward the ends E of the electrodes C₁ to Cz by dielectrophoresis due to the non-uniform electric field L₂ generated in the vicinity of the ends E of the energized electrodes C₁ to Cz. The nucleotide probes X are immobilized so as to bridge the adjacent electrodes (C₁ and C₂, C₂ and C₃, ..., Cy and Cz).

More specifically, in the reaction region R, first ends of the nucleotide probes X in a stretched form migrate to and are attached on the end E₁ of the electrode C₁ by dielectrophoresis due to the non-uniform electric field, and then second ends of the nucleotide probes X migrates to and is attached on the end E₂ of the adjacent electrode C₂ which is energized after the electrode C₁. Thus, the nucleotide probes X are immobilized so as to bridge the adjacent electrodes (C₁ and C₂, C₂ and C₃, ..., Cy and Cz). Reference numeral X' in Fig. 9 denotes the nucleotide probes immobilized on the ends of the electrodes.

### [Sixth Embodiment]

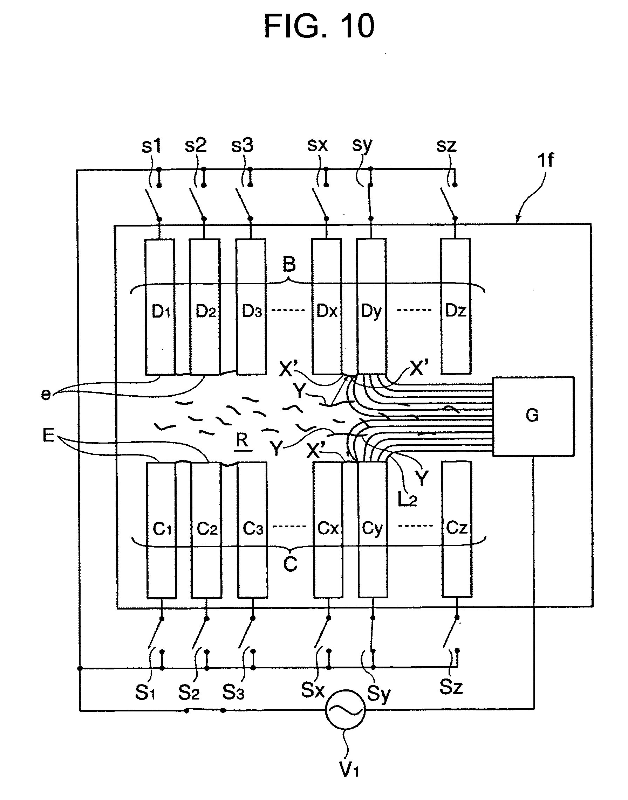

Fig. 10 illustrates a main configuration (reference numeral: 1f) of a detector or a sensor chip according to a sixth embodiment of the present invention.

The detector 1f, like the detector 1e, has a common electrode G in the reaction region R. In the detector 1f, scanning electrodes C consisting of electrodes C₁, C₂, C₃, ..., Cx, Cy, ..., and Cz and scanning electrodes D consisting of electrodes D₁, D₂, D₃, ..., Dx, Dy, ..., and Dz are disposed in two lines. The ends E of the scanning electrodes C oppose the respective ends e of the scanning electrodes D.

The electrodes C₁ to Cz of the scanning electrodes C are connected to a power source V₁ by switching on the respective switches S₁ to Sz one by one. The electrodes D₁ to Dz of the scanning electrodes D are connected to a power source V₁ by switching on the respective switches s₁ to sz one by one. Namely, when the switches S₁ and s₁ are switched on, voltages are applied between the common electrode G and the electrode C₁ and between the common electrode G and the electrode D₁. When switches S₂ and s₂ are switched on, voltages are applied between the common electrode G and the electrode C₂ and between the common electrode G and the electrode D₂. Figs. 2A and 2B show a state in which the switches Sy and sy are switched on and the voltages are applied between the electrodes G and Cy and between the electrodes G and Dy.

### [Seventh Embodiment]

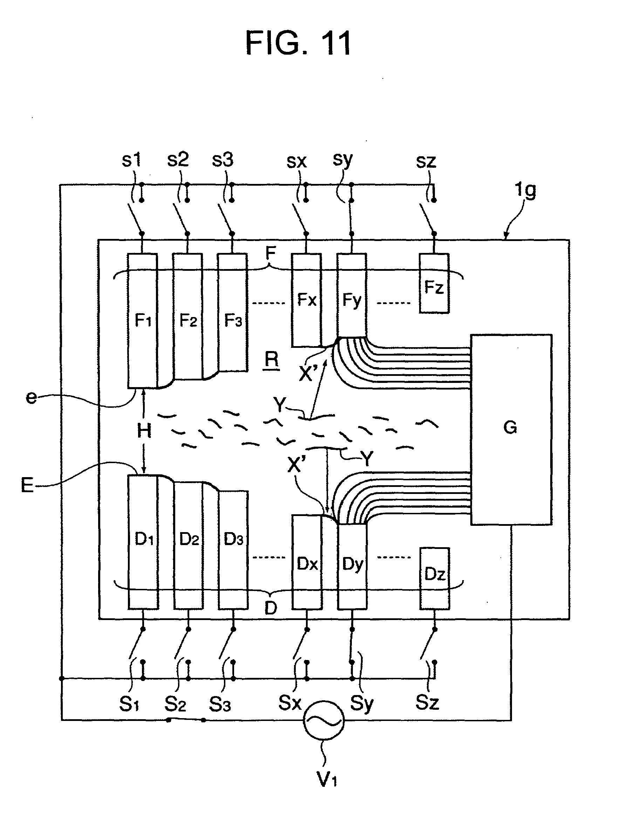

Fig. 11 illustrates a main configuration (reference numeral: 1g) of a detector or a sensor chip according to a seventh embodiment of the present invention.

As with the detectors 1e and 1f, the detector 1g shown in Fig. 3 has a common electrode G in the reaction region R, scanning electrodes D consisting of electrodes D₁, D₂, D₃, ..., Dx, Dy, ..., and Dz, and scanning electrodes F consisting of electrodes F₁, F₂, F₃, ..., Fx, Fy, ..., and Fz. The scanning electrodes D and the scanning electrodes F are aligned in two lines so that each end E of the scanning electrodes D opposes the corresponding end e of the scanning electrodes F.

Each of the electrodes D₁ to Dz of the scanning electrodes D is connected to a power source V₁ by switching on the corresponding switches S₁ to Sz one by one, and each of the electrodes F₁ to Fz of the scanning electrodes F is connected to a power source V₁ by switching on the corresponding switches s₁ to sz one by one. Namely, when the switches S₁ and s₁ are switched on, voltages are applied between the common electrode G and the electrode D₁ and between the common electrode G and the electrode F₁. When switches S₂ and s₂ are switched on, voltages are applied between the common electrode G and the electrode D₂ and between the common electrode G and the electrode F₂. Fig. 11 shows a state in which the switches Sy and sy are switched on and voltages are applied between the electrodes G and Dy and between the electrodes G and Fy.

As shown in Fig. 11, in the seventh embodiment, the distance H between the electrodes D₂ and F₂ is larger than that between the electrodes D₁ and F₁, in the same way, the distance H between the electrodes D₃ and F₃ is larger than that between the electrodes D₂ and F₂, and the distance H between the electrodes Dz and Fz is the largest. With such a configuration, the reaction fields between the electrodes increase stepwise depending on each distance H between the opposing scanning electrodes D and the F increasing stepwise. As a result, the nucleotide probes X can freely migrate by dielectrophoresis to (the end of) the energized electrode in the reaction region R since the nucleotide probes X are free from physical hindrance of the adjacent electrode to which a voltage is not applied. The target nucleotide sequences Y can be subjected to the same effects during dielectrophoresis.

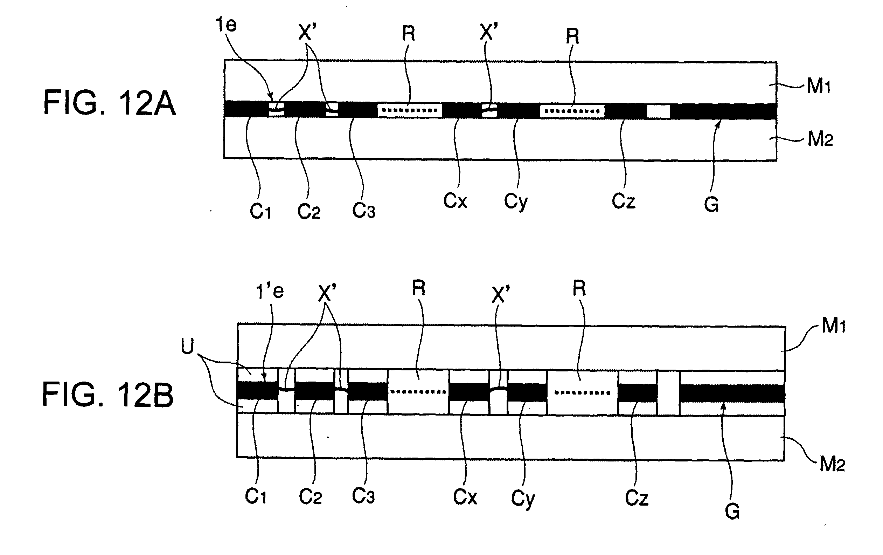

Fig. 12A shows a cross-sectional view taken along line II-II and viewed from the direction of arrows II in Fig. 9. Fig. 12B shows a cross-sectional view of a modification (reference numeral: 1'e) of the detector 1e taken along line II-II and viewed from the direction of arrows II.

As shown in Figs. 12A and 12B, the detectors 1e and 1'e are disposed in a narrow gap between substrates M₁ and M₂ composed of quartz glass, silicon, or a synthetic resin such as polycarbonate or polystyrene. In the detector 1e, the depth (or width) of the reaction region R is the same as the thicknesses of the common electrode G and the scanning electrodes C.

Alternatively, in the detector 1'e shown in Fig. 12B, dielectric layers U may be disposed so as to sandwich the common electrodes G and the scanning electrodes C. In this configuration, the gap between the substrates M₁ and M₂ is increased and the capacity of the reaction region R is increased. In the reaction region R, the scanning electrodes C may be disposed in a plurality of lines in the depth direction of the reaction region R (not shown). The same can be applied to the detectors 1f and 1g.

In the detectors 1e, 1f, and 1g, the target nucleotide sequences Y added to the reaction region R are stretched, in the same manner as the nucleotide probes X, by the non-uniform electric fields L₂ generated between the electrodes G and C (C₁ to Cz), between the electrodes G and D (D₁ to Dz), and between the electrodes G and F (F₁ to Fz). The stretched target nucleotide sequences Y migrate to the nucleotide probes X' (in a stretched form) immobilized on the electrodes C, the electrodes D, and the electrodes F, and efficient hybridizations are conducted without steric hindrance.

Fig. 9 schematically shows a state in which the stretched target nucleotide sequences Y are hybridized to the nucleotide probes X' immobilized between the scanning electrodes Cx and Cy. Fig. 10 schematically shows a state in which the stretched target nucleotide sequences Y are hybridized to the nucleotide probes X' immobilized between the scanning electrodes Cx and Cy and between the scanning electrodes Dx and Dy. Fig. 11 schematically shows a state in which the stretched target nucleotide sequences Y are hybridized to the nucleotide probes X' immobilized between the scanning electrodes Dx and Dy and between the scanning electrodes Fx and Fy.

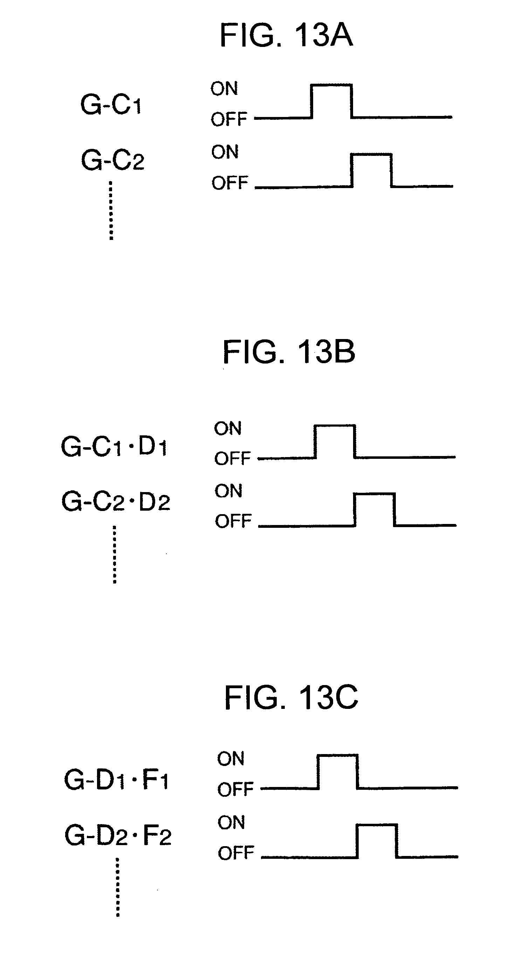

Voltage-applying operations will now be explained with reference to Figs. 13A, 13B, and 13C. Fig. 13A shows an exemplary voltage-applying operation for the detector 1e, Fig. 13B shows an exemplary voltage-applying operation for the detector 1f, and Fig. 13C shows an exemplary voltage-applying operation for the detector 1g.

In the detector 1e shown in Fig. 9, as shown in Fig. 13A, a voltage is applied between the electrodes G and C₁, then between the electrodes G and C₂, ..., one after the other. In the detector 1f shown in Fig. 10, as shown in Fig. 13B, voltages are applied between the electrodes G and C₁ and between G and D₁, then between the electrodes G and C₂ and between G and D₂, ..., one after the other. In the detector 1g shown in Fig. 11, as shown in Fig. 13C, voltages are applied between the electrodes G and D₁ and between G and F₁, then between the electrodes G and D₂ and between G and F₂, ..., one after the other. The duration of each of the voltage applications is appropriately determined.

A series of the voltage-applying operations may be repeated several times, if necessary. Repetition of the series of the voltage-applying operations allows the nucleotide probes X to be tightly immobilized on the scanning electrodes.

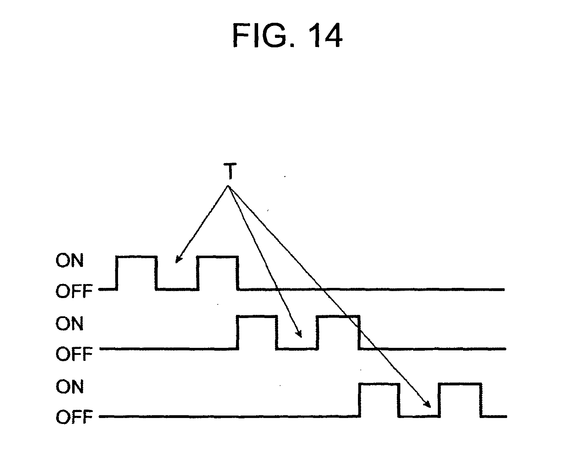

As shown in Fig. 14, when the intermittent voltage applications to the electrodes of the detectors 1e, 1f, and 1g are repeated a desired number of times, the following can be controlled: the timing for immobilizing the nucleotide probes X, stepwise migration of the target nucleotide sequences Y in the reaction region R toward the immobilized nucleotide probes X', backward and forward migration of the target nucleotide sequences Y, and the timing for the reaction.

By providing a duration T when the voltage application between the electrodes is discontinued (see Fig. 14), the duplex-formation reaction, i.e. hybridization, between the target nucleotide sequences Y in a stretched form and the nucleotide probes X' in a stretched form proceeds merely under Brownian motion.

By the above-mentioned voltage-applying operations, the nucleotide probes X' are stretched and are immobilized on the scanning electrodes C, electrodes D, and electrodes F of the detectors 1e, 1f, and 1g, and the target nucleotide sequences Y are stretched in a similar way to the nucleotide probes X'. Therefore, a hydrogen bond is efficiently formed between bases of the nucleotide probes X' and the complementary bases of the target nucleotide sequences Y without steric hindrance. Namely, the target nucleotide sequences Y are efficiently hybridized to the nucleotide probes X'. As a result, the hybridization time can be decreased and the probability of false positivity or false negativity is reduced.

### [Eighth Embodiment]

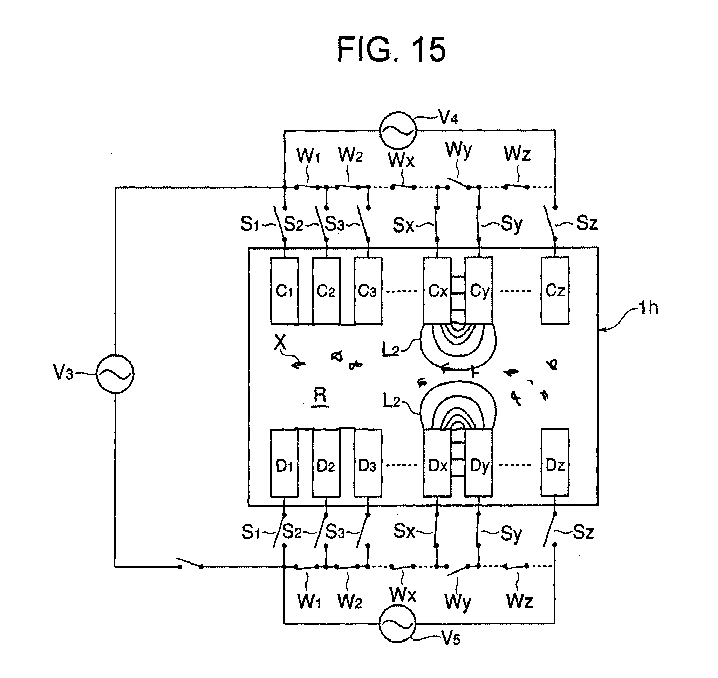

Fig. 15 illustrates a main configuration (reference numeral: 1h) of a detector or a sensor chip according to an eighth embodiment of the present invention.

The detector 1h has a reaction region R for hybridization between the nucleotide probes X and the target nucleotide sequences Y having a base sequence complementary to the nucleotide probes X. In the reaction region R, first scanning electrodes C and second scanning electrodes D are disposed so that each end of the second scanning electrodes D opposes the corresponding end of the first scanning electrodes C.

Voltages are applied between adjacent electrodes of the first scanning electrodes C and between adjacent electrodes of the second scanning electrodes D one after the other. Thus, electric fields are generated one by one. The nucleotide probes are stretched in the electric fields and migrate toward the electrodes applied a voltage by dielectrophoresis, then the nucleotide probes X' are immobilized in a stretched form on the scanning electrodes so as to bridge the adjacent scanning electrodes.

Fig. 15 shows a state in which a voltage is applied between the scanning electrodes Cx and Cy by a power source V₄ and a voltage is applied between the scanning electrodes Dx and Dy by a power source V₅, and thus non-uniform electric fields L₂ are generated.

### [Ninth Embodiment]

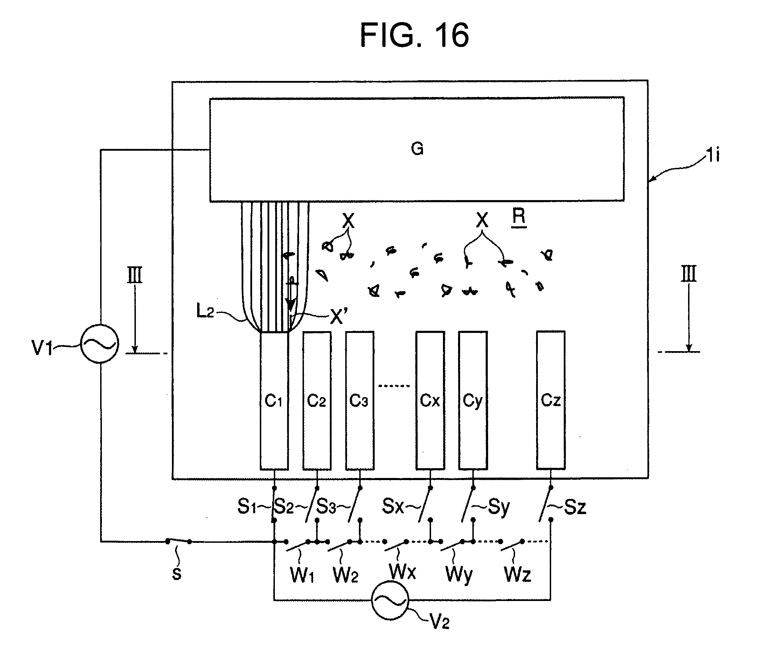

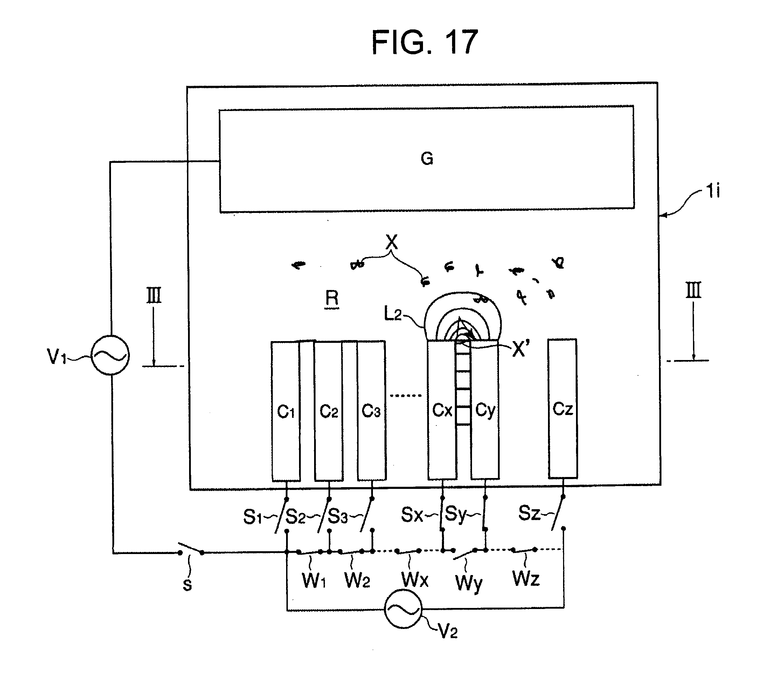

Figs. 16 and 17 illustrate a main configuration (reference numeral: 1i) of a detector or a sensor chip according to a ninth embodiment of the present invention.

The detector 1i has a reaction region R where counter electrodes G and C (consisting of C₁, C₂, C₃, ..., Cx, Cy, and Cz) are disposed. These electrodes can be connected to power sources V₁ and V₂ shown in the drawings.

In the detector 1i, the electrode G is a single rectangular electrode and the electrodes C are scanning electrodes. The scanning electrodes are aligned so as to oppose the electrode G with a predetermined distance. Voltages are applied between the electrode G and the electrodes C₁, C₂, C₃, ..., Cx, Cy, and Cz one by one by switching on and off a switch S, switches S₁, S₂, S₃, ..., Sx, Sy, and Sz, and switches W₁, W₂, W₃, ..., Wx, Wy, and Wz one after the other.

A voltage is sequentially applied to a pair of adjacent scanning electrodes C by switching on and off the switches S₁, S₂, S₃, ..., Sx, Sy, and Sz and the switches W₁, W₂, W₃, ..., Wx, Wy, and Wz one after the other. Electrical connection between the electrode G and the scanning electrodes C generates non-uniform electric fields L₂ at the ends of the scanning electrodes C (see Fig. 16). Electrical connection between the adjacent scanning electrodes C (for example, between the electrodes C₁ and C₂) generates a non-uniform electric field L₂ at each end of the scanning electrodes C (see Fig. 17).

In the ninth embodiment shown in Figs. 16 and 17, first ends of the nucleotide probes X' are immobilized on a scanning electrode (for example, C₁ as shown in Fig. 16) in a stretched form, and then second ends of the nucleotide probes are immobilized on the adjacent scanning electrode (for example, C₂). Thus, the adjacent scanning electrodes (for example, C₁ and C₂) are bridged by the nucleotide probes X'. The target nucleotide sequences (in a stretched form) are then hybridized to the nucleotide probes X' immobilized on and bridging the adjacent electrodes. This process can be applied to any configuration having scanning electrodes and a common electrode G opposing the scanning electrodes.

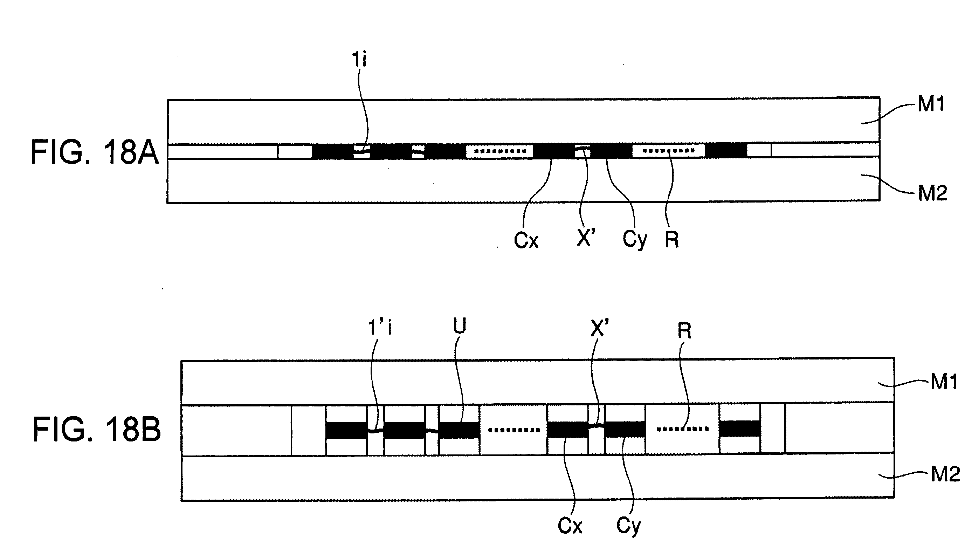

Fig. 18A shows a cross-sectional view taken along line III-III and viewed from the direction of arrows III in Figs. 16 and 17. Fig. 18B shows a cross-sectional view of a modification (reference numeral: 1'i) of the detector 1i shown in Fig. 18A taken along line III-III and viewed from the direction of arrows III.

As shown in Fig. 18A, the detectors 1i and 1'i are disposed in a narrow gap between substrates M₁ and M₂ composed of quartz glass, silicon, or a synthetic resin such as polycarbonate or polystyrene. In the detector 1i, the depth (or width) of the reaction region R is the same as the thicknesses of the electrodes C.

In some cases represented by the detector 1'i shown in Fig. 18B, dielectric layers U may be disposed so as to sandwich each of the electrodes C. In this configuration, the gap between the substrates M₁ and M₂ is increased and the capacity of the reaction region R is increased. In the reaction region R, the scanning electrodes C may be disposed in a plurality of lines (not shown).

Fig. 16 shows a state of the detector 1i (or 1'i) in which the switches S and S₁ shown in the drawing are switched on and thus the power source V₁ applies a high-frequency voltage between the counter electrodes G and C₁.

Application of the high-frequency voltage between the electrodes G and C₁ generates a non-uniform electric field L₂ in the reaction region R. The non-uniform electric field L₂ stretches the nucleotide probes X, which are randomly dispersed in the reaction region R, to be linearly arranged along the non-uniform electric field L₂. The nucleotide probes X then migrate by dielectrophoresis in the non-uniform electric field L₂ and first ends of the nucleotide probes X are immobilized on the end, having a high electric field, of the electrode C₁ in a stretched form.

Next, the high-frequency voltage applied between the electrodes G and C₁ is discontinued by switching off the switch S. At the same time or after switching off the switch S, a voltage is applied between the electrodes C₁ and C₂ from a power source V₂ by switching on switch S₂ and switches W₂ to Wz. By this process, the non-immobilized second ends of the immobilized nucleotide probes X' migrate along the electric lines of force in the non-uniform electric field generated between the electrodes C₁ and C₂, and are immobilized on the end, having a high electric field, of the electrode C₂.

Similarly, each end of the nucleotide probes X is immobilized on the end of the scanning electrode C₂ by switching on the switches S₂ and W₁ and switching off the switches S₁ and W₂, and then the nucleotide probes X' in a stretched form are immobilized between the scanning electrodes C₂ and C₃ by switching off the switches S and W₂ and switching on the switches S₃, W₁, and W₃ to Wz.

As described above, the nucleotide probes X' are immobilized on and bridge the ends of the adjacent scanning electrodes B by sequential voltage application to the electrodes. Fig. 17 shows a state in which the nucleotide probes X' are immobilized on and bridge the adjacent scanning electrodes Cx and Cy.

A high-frequency voltage may be applied between the electrode G and a plurality of electrodes C at the same time for stretching and aligning the nucleotide probes on the ends of these scanning electrodes C (not shown). Voltages are then applied between the adjacent scanning electrodes C to immobilize the nucleotide probes X' and to bridge the adjacent scanning electrodes C by the nucleotide probes X'.

The target nucleotide sequences added to the reaction region R are stretched in the same manner as the nucleotide probes X in the non-uniform electric field generated between the common electrode G and electrodes C. The target nucleotide sequences Y migrate to the ends of the scanning electrodes B by dielectrophoresis, and are efficiently hybridized to the nucleotide probes X' immobilized on the ends of the scanning electrodes B without steric hindrance.

In order to dielectrophorese the target nucleotide sequences toward the scanning electrodes B, an electric field may be applied between the common electrode G and electrodes C, sequentially C₁ to Cx, or electric fields may be simultaneously applied between the electrode G and a plurality of electrodes C having the same potential.

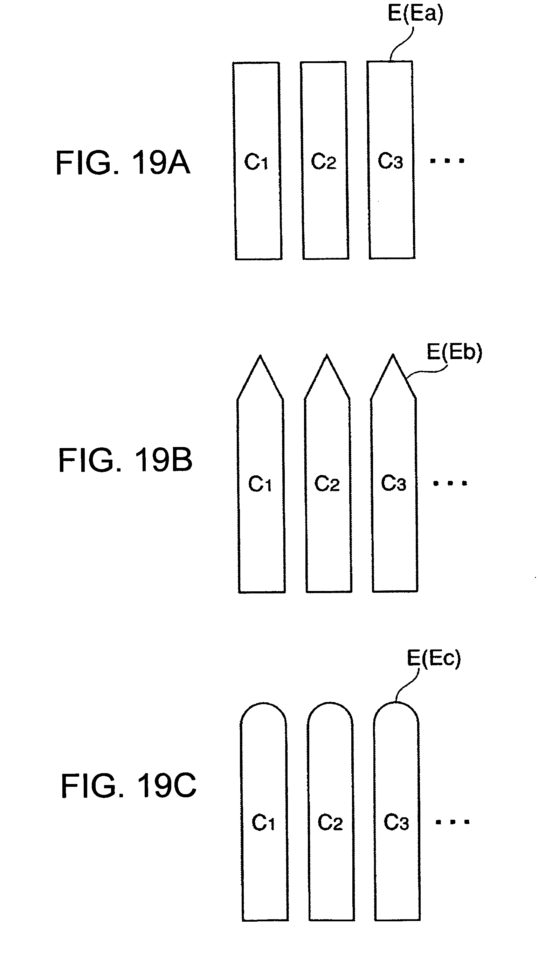

Figs. 19A, 19B, and 19C illustrate typical examples of ends E of the scanning electrodes Cx to Cy, which represent the above-described scanning electrodes (the same is applied to other scanning electrodes D and F). Fig. 19A illustrates scanning electrodes having a rectangular end Ea, Fig. 19B illustrates scanning electrodes having a triangular end Eb, and Fig. 19C illustrates scanning electrodes having a circular end Ec. A scanning electrode having the circular end Ec is preferable because it enhances the generation of a non-uniform electric field L₂ and the immobilization of the nucleotide probes X.

The surfaces of the ends E (Ea, Eb, and Ec) of the scanning electrodes may be treated so that the ends of the nucleotide probes X can be immobilized on the surfaces with a coupling reaction or the like. For instance, a detection surface treated with streptavidin is suitable for immobilizing nucleotide sequences having biotinized ends.

The hybridization is detected by a known method. For example: labeling of the target nucleotide sequences Y with a fluorescent dye or fluorescent intercalator, which specifically binds to base pairs of nucleotides, such as POPO-1 and TOTO-3; irradiation with exciting light; and detection of fluorescent signals with a known detection device.

More specifically, the reaction region R is excited by laser light (for example, blue laser light) and the fluorescence intensity is measured with a detection device (not shown), thus the target nucleotide sequences Y hybridized to the nucleotide probes X' are determined. The fluorescence intensity in each reaction region R can be visualized by converting the analog data to digital data and displaying the distribution of the resulting binding ratios on a computer display.

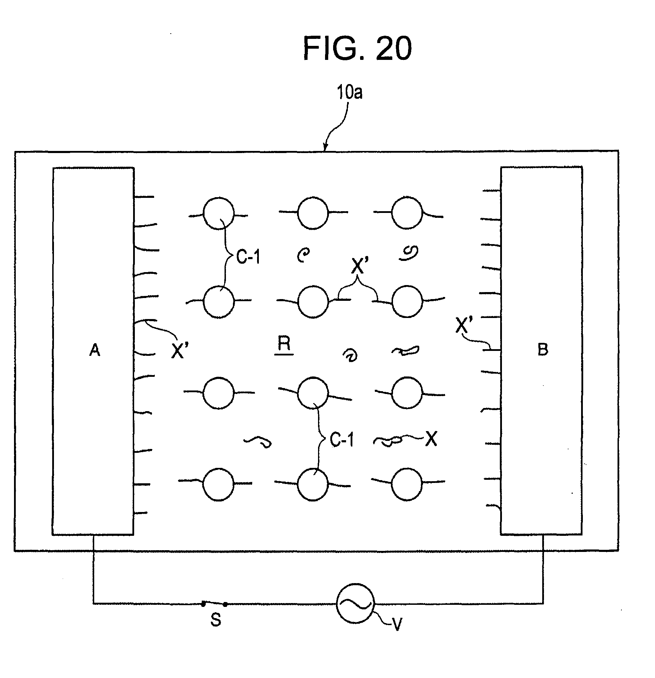

Preferred embodiments of the present invention will now be described with reference to the accompanying drawings. Fig. 20 illustrates a main configuration (reference numeral: 10a) of a hybridization detector (abbreviated to "detector" hereinafter) or a sensor chip according to a tenth embodiment of the present invention.

The detector 10a has a reaction region R. The reaction region R is a reservoir for adding solutions containing nucleotide probes X denoted by X in Fig. 20 and containing target nucleotide sequences Y (not shown), and the reaction region R is a portion or space where hybridizations are performed.

Counter electrodes A and B are disposed at the reaction region R and are connected to a power source V (shown in the drawing) by a switch S. The counter electrodes A and B are aligned so that the end faces of the electrodes are in parallel with each other. A plurality of floating-potential electrodes C-1 is linearly distributed in the lengthwise and widthwise directions between counter electrodes (see Fig. 20) and is not connected to the power source V. Although the drawn floating-potential electrodes C-1 are circular, their shape is not limited to this. As long as a non-uniform electric field is generated, the floating-potential electrodes can be designed in any other shape such as a polygon or ellipse.

Preferably, the dimensions of each surface of the floating-potential electrodes C-1 are smaller than those of the counter electrodes A and B. Since the floating-potential electrodes C-1 have narrower surfaces, electric fields can be concentrated toward the surfaces of the floating-potential electrodes C-1, and non-uniform electric fields can be readily generated.

Fig. 20 shows a state when a voltage is applied between the counter electrodes A and B by switching on the switch S. Reference numeral X shown in the reaction region R denotes the nucleotide probes that are still randomly coiled or folded, and reference numeral X' denotes the nucleotide probes that are stretched and are immobilized on the electrode surfaces.

The detector 10a is disposed in a narrow gap between substrates composed of quartz glass, silicon, or a synthetic resin such as polycarbonate or polystyrene (not shown). In the detector 10a, the depth (or width) of the reaction region R is the same as the thicknesses of the electrodes such as the counter electrodes A and B. In some cases, dielectric layers may be disposed so as to sandwich the counter electrodes A and B and the rest. Such a configuration increases the gap between the substrates and the capacity of the reaction region R (the same is applied to an eleventh embodiment described below).

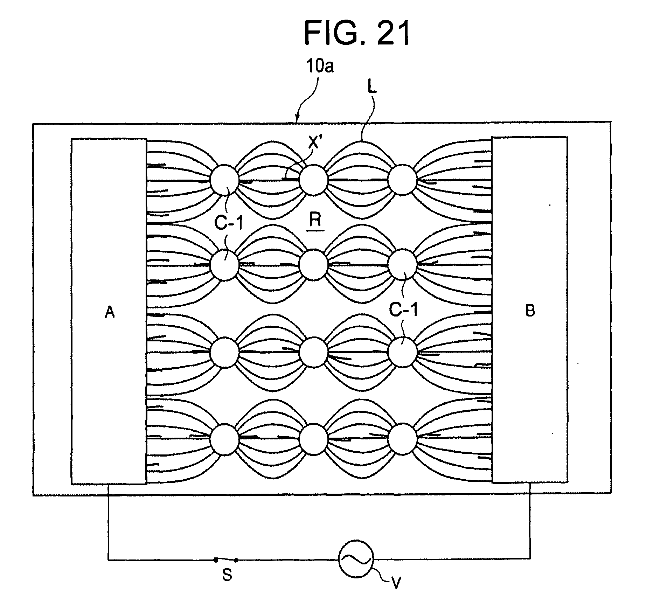

Fig. 21 illustrates a state of the detector 10a in which a high-frequency voltage is applied between the counter electrodes A and B from the power source V by switching on the switch S shown in the drawing to generate electric fields L shown by electric lines of force in the reaction region R. The high-frequency voltage, applied between the counter electrodes A and B, generates non-uniform electric fields denoted by reference numeral L in the reaction region R.

Namely, the voltage applied to the counter electrodes A and B generates non-uniform electric fields L at the counter electrodes A and B and at the partial surfaces, where the electric fields are concentrated, of the floating-potential electrodes C-1 (see Fig. 21). The nucleotide probes X, which are randomly dispersed in the reaction region R, is stretched and migrate by the non-uniform electric fields L along the non-uniform electric fields L.

The nucleotide probes X then migrate to the surfaces, having high electric fields, of the electrodes A, B, and C-1 by dielectrophoresis and each end of the nucleotide probes X is immobilized in a stretched form. Figs. 20 and 21 show a state in which the nucleotide probes X' in a stretched form are immobilized on the surface of electrodes A, B, and C-1.

Preferably, the parameters of the electric field between the counter electrodes A and B are about 1 × 10⁶ V/m and about 1 MHz (see Masao Washizu and Osamu Kurosawa: "Electrostatic Manipulation of DNA in Microfabricated Structures", IEEE Transaction on Industrial Application Vol. 26, No. 26, p.1165-1172 (1990)).

The target nucleotide sequences Y are subsequently added to the reaction region R. In the same manner as the nucleotide probes X', the target nucleotide sequences Y are stretched by the non-uniform electric fields L between the counter electrodes A and B and migrate by dielectrophoresis to the surfaces, having high electric fields, of the electrodes A, B, and C-1.

The migrating target nucleotide sequences are efficiently hybridized to the nucleotide probes X', which are already immobilized on the surfaces in a stretched form, without steric hindrance.

The surfaces or ends of the counter electrodes A and B and the floating-potential electrodes C-1 may be treated so that the ends of the nucleotide probes X' are immobilized on the surfaces or the ends by a coupling reaction or the like. For instance, an electrode surface treated with streptavidin is suitable for immobilizing nucleotide sequences having biotinized ends.

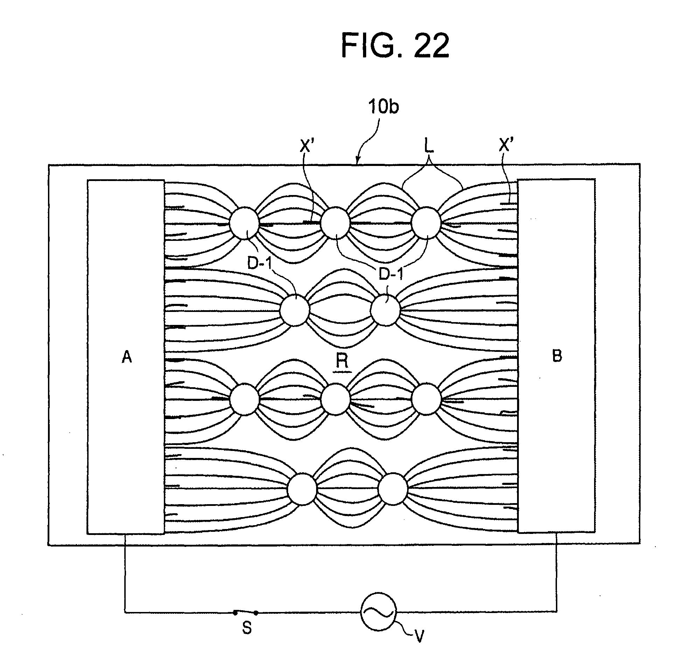

With reference to Fig. 22, the configuration (reference numeral: 10b) of a detector or a sensor of an eleventh embodiment of the present invention will now be explained.

The detector 10b is the same as the detector 10a in that the detector 10b has a reaction region R, and counter electrodes A and B are aligned in parallel with each other at the reaction region R and can be connected to a power source V. A plurality of floating-potential electrodes D-1 is disposed between the counter electrodes A and B and is not connected to the power source V.

The floating-potential electrodes D-1 of the eleventh embodiment and the floating-potential electrodes C-1 of the tenth embodiment are the same in that both of them are distributed in the reaction region R, but are different in that the floating-potential electrodes D-1 of the detector 10b are alternately arrayed (see Fig. 22) whereas the floating-potential electrodes C-1 of the detector 10a are arrayed linearly in the lengthwise and widthwise directions (see Figs. 20 and 21). In Fig. 22, although the drawn floating-potential electrodes D-1 are circular, their shape is not limited to this. As long as a non-uniform electric field is generated, the floating-potential electrodes can be designed in any other shape such as a polygon or ellipse.

In the tenth and eleventh embodiments, the floating-potential electrodes C-1 and the floating-potential electrodes D-1 are arrayed with an interval, i.e. in a matrix layout, in the reaction region R. As a result, a large number of areas where electric lines of force are concentrated can be generated in the reaction region R, and the nonuniformity of electric fields can be enhanced in the reaction region R.

The floating-potential electrodes C-1 distributed in the reaction region R allow the proceeding of hybridization in the entire reaction region R without restricting free migration of the nucleotide sequences. Therefore, the detection sensitivity is improved because the area for hybridization is expanded to the entire reaction region R.

The distances between the adjacent floating-potential electrodes C-1 and between the adjacent floating-potential electrodes D-1 are not limited, but preferably, each distance is larger than twice the molecular length of the nucleotide probes X' in a stretched form. In such a configuration, the nucleotide probes X' can freely migrate between the adjacent floating-potential electrodes C-1 and C-1 or between the adjacent floating-potential electrodes D-1 and D-1, and interference and steric hindrance between the immobilized nucleotide probes X' are avoided.

On the other hand, when the distance between each of the floating-potential electrodes C-1 is smaller than the length of the nucleotide probes X' in a stretched form, the nucleotide probes X' can be immobilized so as to bridge the adjacent floating-potential electrodes C-1 and C-1.

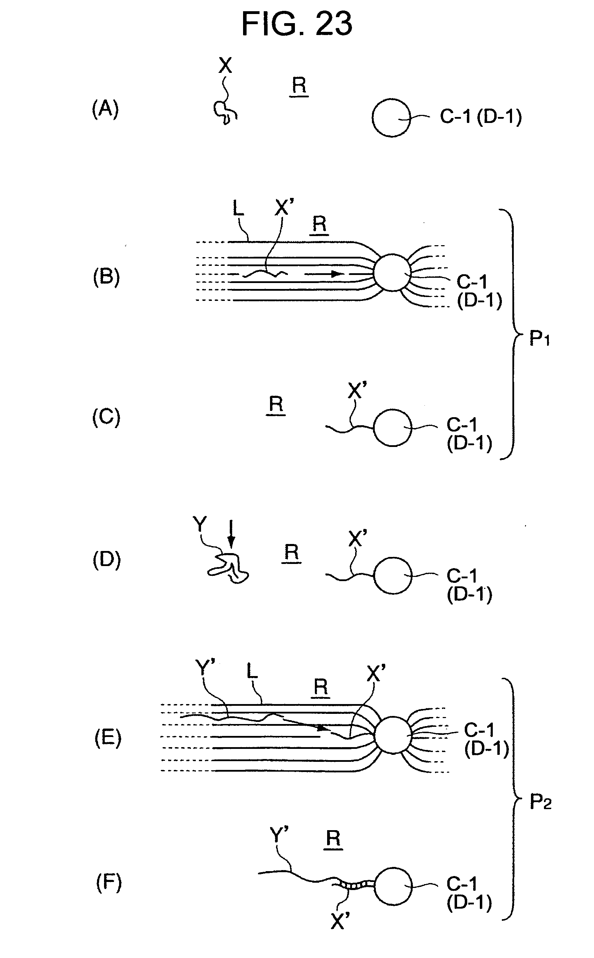

With reference to Fig. 23, an embodiment of a hybridization process of the present invention will now be described. Fig. 23 shows a brief flowchart of the process.

Fig. 23(A) illustrates a stage, immediately after the nucleotide probe X is added to the reaction region R, in which the nucleotide probe X is still in a folded form. In this stage, the nucleotide probe is not immobilized on the floating-potential electrode C-1 (or D-1).

Figs. 23(B) and 23(C) illustrate a first step P₁ of the hybridization process of the present invention. Fig. 23(B) illustrates a stage in which a voltage is applied between the counter electrodes A and B shown in Figs. 20 to 22, a non-uniform electric field L is generated, i.e. electric lines of force are concentrated at the surface of the floating-potential electrode C-1 (or D-1), the nucleotide probe X is stretched along the electric lines of force, and the nucleotide probe X migrates toward the floating-potential electrode C-1 (or D-1). Fig. 23(C) illustrates the next stage in which first end of the nucleotide probe X' in a stretched form is immobilized on the floating-potential electrode C-1 (or D-1).

Fig. 23(D) illustrates a stage in which a target nucleotide sequence Y is added to the reaction region R. In this stage, the supply of the voltage to the counter electrodes A and B is discontinued and the target nucleotide sequence Y is folded. The supply of the voltage to the counter electrodes A and B may be continued during this stage for addition of the target nucleotide sequences Y to the reaction region R.

Figs. 23(E) and 23(F) illustrate a second step P₂ of the hybridization process of the present invention. Fig. 23(E) illustrates a stage in which a voltage is applied again between the counter electrodes A and B to generate a non-uniform electric field L, i.e. electric lines of force are concentrated at the surface of the floating-potential electrode C-1 (or D-1), the target nucleotide sequence Y is stretched along the electric lines of force, and the target nucleotide sequence Y migrates toward the nucleotide probe X'.

Fig. 23(F) illustrates a stage in which the voltage is not applied, and the target nucleotide sequence Y and the nucleotide probe X', both are stretched, are subjected to Brownian motion and are hybridized.

In the hybridization process of the present invention, the counter electrodes A and B may be continuously energized over the stages shown in Figs. 23(B) to 23(E) by switching on the switch S or may be intermittently energized by switching on and off the switch S.

The intermittent supply of a voltage repeated a desired number of times can control the timing for immobilizing the nucleotide probes X' on the electrodes, stepwise migration of the target nucleotide sequences Y in the reaction region R toward the immobilized nucleotide probes X', backward and forward migration of the target nucleotide sequences Y, and the timing for the reaction.

The discontinuation of the voltage application to the electrodes (especially, in the stage shown in Fig. 23(F)) allows the duplex-formation reaction, i.e. hybridization, between the target nucleotide sequence Y in a stretched form and the nucleotide probe X' in a stretched form proceeds merely under Brownian motion.

By the above-mentioned process, a hydrogen bond is formed without steric hindrance between bases of the nucleotide probe X' immobilized on the electrode in a stretched form and the complementary bases of the target nucleotide sequence Y stretched in the same manner as the nucleotide probe X', i.e. hybridization is efficiently performed. As a result, the hybridization time can be decreased and the probability of false positivity or false negativity is reduced.

The hybridization is detected by a known method. For example: labeling of the target nucleotide sequences Y with a fluorescent dye or fluorescent intercalator, which specifically binds base pairs of nucleotides, such as POPO-1 and TOTO-3; irradiation with exciting light; and detection of fluorescent signals with a known detection device.

More specifically, the reaction region R is excited by laser light (for example, blue laser light) and the fluorescence intensity is measured with a detection device (not shown), thus the hybridization between the target nucleotide sequences Y and the nucleotide probes X' is determined. The fluorescence intensity in each reaction region R can be visualized by converting the analog data to digital data and displaying the distribution of the resulting binding ratios on a computer display. In the present invention, the method for detecting hybridization is not limited.

According to the present invention, the nucleotide probes are stretched and are then arrayed and immobilized (between scanning electrodes) in a reaction region for hybridization on a surface of a sensor chip such as a DNA chip. As a result, improvement in hybridization efficiency, reduction in reaction time, and prevention of false positivity and false negativity are ensured.

According to the present invention, the nucleotide probes are stretched and then immobilized in an entire reaction region for hybridization on a surface of a sensor chip such as a DNA chip. As a result, improvement in hybridization efficiency, reduction in reaction time, improvement in sensitivity, and prevention of false positivity and false negativity are ensured.

## Claims

1. A hybridization detector comprising a reaction region for hybridization between nucleotide probes and target nucleotide sequences having a base sequence complementary to the nucleotide probes, wherein the reaction region has a configuration for stretching the nucleotide probes by an electric field and for immobilizing the nucleotide probes by dielectrophoresis on scanning electrodes arrayed in the reaction region.

2. A sensor chip comprising the hybridization detector of claim 1.

3. A sensor chip comprising:
a reaction region for hybridization between nucleotide probes and target nucleotide sequences having a base sequence complementary to the nucleotide probes;
counter electrodes generating a uniform electric field for stretching the nucleotide probes in the reaction region;
scanning electrodes arrayed in the reaction region, the electrodes being capable of being energized; and
means for dielectrophoresis of the nucleotide probes stretched by the counter electrodes toward a pair of the adjacent scanning electrodes by a non-uniform electric field generated by applying a voltage between the adjacent scanning electrodes, and immobilizing the nucleotide probes in a stretched form so as to bridge the adjacent scanning electrodes.

4. The sensor chip according to claim 3, wherein target nucleotide sequences are hybridized to the nucleotide probes immobilized between the scanning electrodes by dielectrophoresis of the target nucleotide sequences stretched in the uniform electric field toward the scanning electrodes.

5. The sensor chip according to claim 3, wherein the scanning electrodes have circular or polygonal ends.

6. The sensor chip according to claim 3, wherein the counter electrodes are disposed so as to oppose and be in parallel with each other.

7. The sensor chip according to claim 2, wherein the electric fields generated by the counter electrodes and the scanning electrodes are of alternate current.

8. A sensor chip comprising:
a reaction region for hybridization between nucleotide probes and target nucleotide sequences having a base sequence complementary to the nucleotide probes;
a common electrode disposed in the reaction region;
scanning electrodes formed of a plurality of electrodes aligned in parallel; and
means for generating electric fields by sequentially applying a voltage between the common electrode and each of the scanning electrodes, dielectrophoresis of the nucleotide probes in the reaction region toward the energized scanning electrodes while the nucleotide probes are being stretched by the electric fields, and immobilizing the nucleotide probes in a stretched form so as to bridge the scanning electrodes.

9. The sensor chip according to claim 8 comprising the common electrode and the scanning electrodes, wherein the scanning electrodes are aligned in two lines so that each end of the scanning electrodes opposes each other.

10. The sensor chip according to claim 9, wherein the scanning electrodes are disposed so that the distances between the opposing scanning electrodes increase stepwise in the direction that a voltage is sequentially applied.

11. The sensor chip according to claim 8, wherein the target nucleotide sequences in a stretched form are hybridized to the nucleotide probes immobilized between the scanning electrodes by sequentially applying a voltage between the common electrode and the scanning electrodes, and dielectrophoresis of the target nucleotide sequences in the reaction region toward the energized scanning electrodes while the target nucleotide sequences are being stretched.

12. The sensor chip according to claim 8, wherein the scanning electrodes have circular or polygonal ends.

13. The sensor chip according to claim 8, wherein the electric fields generated by the common electrode and the scanning electrodes are of alternate current.

14. A sensor chip comprising:
a reaction region for hybridization between nucleotide probes and target nucleotide sequences having a base sequence complementary to the nucleotide probes;
first scanning electrodes arrayed in the reaction region;
second scanning electrodes arrayed so that the ends of the second scanning electrodes oppose the respective ends of the first scanning electrodes; and
means for generating electric fields by sequentially applying a voltage between the adjacent electrodes of the first scanning electrodes and between the adjacent electrodes of the second scanning electrodes, dielectrophoresis of the nucleotide probes toward the energized scanning electrodes while the nucleotide probes are being stretched by the electric fields, and immobilizing the nucleotide probes in a stretched form so as to bridge the scanning electrodes.

15. The sensor chip according to claim 14, wherein the target nucleotide sequences stretched in the same manner as the nucleotide probes are hybridized to the nucleotide probes immobilized between the scanning electrodes by dielectrophoresis of the stretched target nucleotide sequences toward the energized scanning electrodes.

16. The sensor chip according to claim 14, wherein the first scanning electrodes and the second scanning electrodes have circular or polygonal ends.

17. The sensor chip according to claim 14, wherein the electric fields generated by the first scanning electrodes or generated by the second scanning electrodes are of alternate current.

18. A sensor chip comprising:
a reaction region for hybridization between nucleotide probes and target nucleotide sequences having a base sequence complementary to the nucleotide probes;
a common electrode disposed in the reaction region;
scanning electrodes arrayed so that the ends of the scanning electrodes oppose the common electrode;
means for generating electric fields by sequentially applying a voltage between the common electrode and each electrode of the scanning electrodes and for dielectrophoresis of the nucleotide probes toward the energized scanning electrodes while the nucleotide probes are being stretched by the electric fields; and
means for immobilizing the nucleotide probes in a stretched form so as to bridge the scanning electrodes by sequentially applying a voltage between the adjacent scanning electrodes.

19. The sensor chip according to claim 18, wherein the target nucleotide sequences stretched in the same manner as the nucleotide probes are hybridized to the nucleotide probes immobilized between the scanning electrodes by dielectrophoresis of the stretched target nucleotide sequences toward the energized scanning electrodes.

20. The sensor chip according to claim 18, wherein the scanning electrodes have circular or polygonal ends.

21. The sensor chip according to claim 18, wherein the electric fields generated between the common electrode and the scanning electrodes and between the scanning electrodes are of alternate current.

22. A method of hybridization using a hybridization detector comprising a reaction region for hybridization between nucleotide probes and target nucleotide sequences having a base sequence complementary to the nucleotide probes and scanning electrodes arrayed in the reaction region, the method comprising the steps of:
stretching the nucleotide probes in the reaction region by an electric field and immobilizing the stretched nucleotide probes on the scanning electrodes by dielectrophoresis; and
hybridizing the target nucleotide sequences to the immobilized nucleotide probes.

23. The method of hybridization according to claim 22, the method further comprising the steps of:
immobilizing first ends of the nucleotide probes on a selected single scanning electrode and subsequently immobilizing second ends of the nucleotide probes on the adjacent scanning electrode so that the nucleotide probes bridge the adjacent scanning electrodes.

24. A hybridization detector comprising:
a reaction region for hybridization between nucleotide probes and target nucleotide sequences having a base sequence complementary to the nucleotide probes;
counter electrodes disposed in the reaction region; and
floating-potential electrodes being dispersed between the counter electrodes.

25. The hybridization detector of claim 24, wherein the floating-potential electrodes have a shape being capable of generating a non-uniform electric field.

26. The hybridization detector according to claim 24, wherein each surface of the floating-potential electrodes is smaller than that of the counter electrodes.

27. The hybridization detector according to claim 24, wherein the surfaces of the floating-potential electrodes are treated for immobilizing the nucleotides probes.

28. The hybridization detector according to claim 24, wherein the counter electrodes are aligned in parallel with each other.

29. The hybridization detector according to claim 24, wherein the electric field generated by the counter electrodes is of alternate current.

30. A sensor chip comprising at least the hybridization detector of claim 24.

31. A method of hybridization using a hybridization detector comprising a reaction region for hybridization between nucleotide probes and target nucleotide sequences having a base sequence complementary to the nucleotide probes and counter electrodes disposed in the reaction region and a plurality of floating-potential electrodes aligned between the counter electrodes, the method comprising the steps of:
stretching the nucleotide probes in the reaction region by applying a voltage to the counter electrodes and immobilizing the stretched nucleotide probes on the surfaces of the floating-potential electrodes by dielectrophoresis in non-uniform electric fields generated at the counter electrodes and at the partial surfaces of the floating-potential electrodes; and
stretching the target nucleotide sequences in the reaction region by applying a voltage to the counter electrodes, and hybridizing the stretched target nucleotide sequences to the stretched nucleotide probes immobilized on the surfaces of the floating-potential electrodes.
